# EUROPEAN PATENT SPECIFICATION

(11) **EP 2 538 961 B1**
(45) Date of publication and mention of the grant of the patent: **12.10.2016**
(21) Application number: 11746962.7
(22) Date of filing: 23.02.2011
(51) Int. Cl.: A61K 38/16, A61K 38/07, A61K 38/10, A61K 31/4412, A61P 43/00

(54) **INHIBITION OF NECROSIS**
HEMMUNG VON NEKROSE
INHIBITION DE LA NÉCROSE

(30) Priority: 24.02.2010 US 307647 P
(43) Date of publication of application: 02.01.2013
(73) Proprietor: Ben Gurion University Of The Negev Research And Development Authority, 84105 Beer Sheva (IL); Mor Research Applications Ltd., 69710 Tel Aviv (IL)
(72) Inventor: PAROLA, Abraham, 84965 Omer (IL); NATHAN, Ilana, 84965 Omer (IL); KASHER, Ron, 84990 Sde Boker (IL); LERNER YARDENI, Jenny, 84719 Beer Sheva (IL); COHEN, Aviv, 43217 Raanana (IL)
(74) Representative: Boult Wade Tennant
(86) International application number: PCT/IL2011/000182
(87) International publication number: WO 2011/104708

(56) References cited:
- EP-A1- 1 221 480
- WO-A1-2010/029537
- US-A- 5 846 984
- US-A1- 2005 233 413
- US-A1- 2005 288 368
- US-A1- 2009 053 203
- US-A1- 2009 069 242
- COHEN A ET AL: "Synthesis of Humanin and its Derivatives for Use as an Anti-Necrotic Agent", BIOPOLYMERS, JOHN WILEY & SONS, INC, US, vol. 96, no. 4, 1 January 2011 (2011-01-01), page 470, XP009172934, ISSN: 0006-3525
- MATSUOKA MASAAKI ET AL: "Humanin and the Receptors for Humanin", MOLECULAR NEUROBIOLOGY, HUMANA PRESS, US, vol. 41, no. 1, 20 November 2009 (2009-11-20), pages 22-28, XP009172930, ISSN: 0893-7648, DOI: 10.1007/S12035-009-8090-Z [retrieved on 2009-12-09]
- MATSUYAMA MASAHIDE ET AL: "The effect of neutrophil elastase inhibitor on acute tubular necrosis after renal ischemia-reperfusion injury", MOLECULAR MEDICINE REPORTS, SPANDIDOS PUBLICATIONS, GR, vol. 1, no. 4, 1 July 2008 (2008-07-01), pages 489-492, XP009172955, ISSN: 1791-2997
- XU XINGSHUN ET AL: "Humanin is a novel neuroprotective agent against stroke.", STROKE; A JOURNAL OF CEREBRAL CIRCULATION OCT 2006, vol. 37, no. 10, October 2006 (2006-10), pages 2613-2619, XP002713880, ISSN: 1524-4628
- KARIYA S ET AL: "HUMANIN INHIBITS CELL DEATH OF SERUM-DEPRIVED PC12H CELLS", NEUROREPORT, LIPPINCOTT WILLIAMS & WILKINS, UK, vol. 13, no. 6, 7 May 2002 (2002-05-07), pages 903-907, XP009016247, ISSN: 0959-4965, DOI: 10.1097/00001756-200205070-00034
- XU X ET AL: "Synergistic protective effects of humanin and necrostatin-1 on hypoxia and ischemia/reperfusion injury", BRAIN RESEARCH, ELSEVIER, AMSTERDAM, NL, vol. 1355, 8 October 2010 (2010-10-08), pages 189-194, XP027276139, ISSN: 0006-8993 [retrieved on 2010-08-01]

## Description

### FIELD OF INVENTION

This invention is directed to, *inter alia*, a composition including Humanin or a derivative of Humanin with or without a necrosis inhibitor effective in inhibiting necrosis or protecting a cell or a tissue exposed to pro-necrotic factors.

### BACKGROUND OF THE INVENTION

Necrosis has been used for a very long time (approximately 2000 years) to mean drastic tissue changes visible to the naked eye. It is important, both conceptually and didactically, to preserve this usage.

Necrosis is considered to be a unique process of death of cells and living tissue, distinguished from apoptotic programmed cell death. Necrosis is characterized by cell swelling, chromatin digestion, and disruption of the plasma and organelle membranes. Latter stages of necrosis are characterized by extensive DNA hydrolysis, vacuolation of the endoplasmic reticulum, organelle breakdown, and cell lysis. The release of intracellular contents after plasma membrane rupture is the cause of inflammation seen with necrosis. Necrosis has long been viewed as an accidental pathological mode of cell death. Recent studies have presented several lines of evidence indicating that necrosis is a regulated process.

Thus, Apoptosis and necrosis significantly differ. Apoptosis unlike necrosis is energy dependent. Under a microscope it is evident that an apoptotic cell undergoes cell shrinkage wherein necrosis results in cell swelling. While membrane integrity is maintained during early stages of apoptosis, in necrosis the integrity of the cell membrane is lost. Apoptosis is characterized by caspases activation, and DNA fragmentation, however both processes that are absent in necrosis.

In contrast to apoptosis, cleanup of cell debris by phagocytes of the immune system is generally more difficult, as the regulated necrotic pathway generally does not provide specific cell signals for resident or recruited phagocytes to dispose of the necrotic cells and byproducts thereof. The immune system, as a consequence of the lack of appropriate specific signals is less capable of locating necrotic cells and tissue and thereby disposing of the noxious products.

There are many causes of necrosis including prolonged exposure to injury, ischemia, infection, cancer, infarction, poisons, venoms and inflammation. Necrosis can also arise from lack of proper care to a wound site.

Necrosis also plays a part in the pathology of several severe diseases including myocardial infarction, brain stroke, liver cirrhosis and other potentially lethal diseases. Several existing therapies for necrosis, such as early and aggressive surgical debridement and exploration of necrotic tissue, hyperbaric oxygen therapy, administration of antibiotics, anti-inflammatory drugs and intravenous immunoglobulin are used with mixed success. An ideal treatment for inhibiting and/or treating necrosis is unavailable and a significant morbidity and mortality is attributable to complications of necrosis.

The prevalence of heart failure continues to increase in the Western world, making it one of the biggest killers in this region. It is characterized by loss of the muscle cells of the heart (cardiomyocytes). Recent studies indicate that cell death by necrosis has a significant role in the cardiomyocyte loss that accompanies heart failure.

### SUMMARY OF THE INVENTION

In one embodiment, disclosed herein is use of a therapeutically effective amount of a composition comprising peptide comprising an amino acid sequence selected from the group consisting of SEQ ID NOs: 1-6, wherein the effective amount inhibits tissue necrosis for treating a subject suffering from a disease characterized by tissue necrosis.

In another embodiment, further disclosed herein is use of a composition comprising a peptide comprising an amino acid sequence selected from the group consisting of SEQ ID NOs: 1-6 for inhibiting necrosis in a cell.

In another embodiment, further disclosed herein is use of a composition comprising a therapeutically effective amount of a peptide comprising an amino acid sequence selected from the group consisting of SEQ ID NOs: 1-6 for preventing pancreatitis in a subject.

In another embodiment, further disclosed herein is use of a therapeutically effective amount of a peptide comprising an amino acid sequence selected from the group consisting of SEQ ID NOs: 1-6 for inhibiting necrosis in a heart of a subject.

In another embodiment, further disclosed herein is use of a composition comprising:
(a) a necrosis inhibitor; and (b) Humanin or a peptide comprising an amino acid sequence selected from the group consisting of SEQ ID NOs: 1-6.

### BRIEF DESCRIPTION OF THE DRAWINGS

Figure 1. Are bar graphs showing the effect of 10 µM humanin (HN) and its derivatives on KCN-induced necrosis in U-937 cells. LDH release was measured after 7 hours (A) % LDH release with the crude data; (B) The amount of LDH released of control cells was subtracted from all treatments, P*<0.02.
Figure 2. Are bar graphs showing the effect of 10 µM HN and its derivatives on KCN-induced necrosis in PC12 cells. LDH release was measured after 5 hours (A) % LDH release with the crude data; (B) The amount of LDH released from control cells was subtracted from all treatments, P*<0.05.
Figure 3. Is a bar graph showing the effect of different HN17 concentrations on KCN-induced necrosis in PC12 cells as assessed by the determination of LDH release. PC12 cells were treated with or without 7mM KCN for 5 hours in the presence or absence of different concentrations of HN17 (1-30µM), and then LDH release from the cells was determined, P*<0.04.
Figure 4. Are bar graphs showing the effect of 10 µM HN and its derivatives on staurosporine/oligomicyn induced necrosis in PC12 cells. LDH release was measured after 4 hours (A) % LDH release with the crude data; (B) The amount of LDH released from control cells was subtracted from all treatments, P*<0.05.
Figure 5. Is a bar graph showing the effect of 10 µM HN and its derivatives on staurosporine/ oligomicyn induced necrosis in PC12 cells. Necrosis percentage as assessed by ethydium bromide and acridine orange double staining after 4 hours, P*<0.04.
Figure 6. Are bar graphs showing the effect of 10 µM HN and its derivatives on KCN-induced necrosis in different NSC34 cell types. Different NSC34 cell types were exposed to 15mM KCN for 5 hours. Cell death was determined by measuring LDH release:(A) NSC34 cells without plasmid-control;(B) NSC34 cells with SOD1 mutant plasmid, P*<0.05.
Figure 7. Is a photograph of a 96 wells plate. The results show the amount of LDH release under necrosis inducing conditions (KCN) with or without the rescue compounds: Humanin derivatives (the peptides described herein) and Elastase inhibitor III. Dark colored wells indicate high LDH release which is equivalent to high levels of necrotic cell death.
Figure 8. Is a bar graph summarizing the necrosis (induced by KCN) rescue effects as measured by the reduction of LDH release of various concentration of Humanin derivative encoded by SEQ ID NO: 3, Elastase inhibitor III, or their combination.
Figure 9. Is a bar graph summarizing the necrosis (induced by KCN) rescue effects as measured by the reduction of LDH release of various concentration of Humanin derivative encoded by SEQ ID NO: 3, Mimosine, or their combination.

### DETAILED DESCRIPTION OF THE INVENTION

In one embodiment, disclosed herein is use of a therapeutically effective amount of a composition comprising: a peptide comprising an amino acid sequence selected from the group consisting of SEQ ID NOs: 1-6 for treatment of a subject suffering from tissue necrosis. In another embodiment, the use comprises a therapeutically effective amount of a composition comprising: (a) a peptide comprising an amino acid sequence selected from the group consisting of SEQ ID NOs: 1-6; and (2) a necrosis inhibitor such as but not limited to an inhibitor of neutrophil Elastase or Mimosine. In another embodiment, the phrase "a peptide comprising an amino acid sequence selected from the group consisting of SEQ ID NOs: 1-6" comprises any one peptide and/or any combination of peptides of SEQ ID NOs: 1-6. In another embodiment, inhibitor of neutrophil Elastase is Elastase inhibitor III. In another embodiment, a necrosis inhibitor is Mimosine. In another embodiment, Elastase inhibitor III is HLE Inhibitor MeOSuc-AAPV-CMK. In another embodiment, a peptide described herein comprises an amino acid sequence selected from the group consisting of SEQ ID NOs:1-6

In another embodiment, use is of therapeutically effective amount of the Humanin peptide or a fragment thereof or a homologue or variant thereof alone or in combination with a necrosis inhibitor such as an inhibitor of neutrophil Elastase or Mimosine. In another embodiment, the effective amount inhibits tissue necrosis. In another embodiment, the subject is afflicted with a disease characterized in that affected tissue is undergoing necrosis as opposed to apoptosis. In another embodiment, a therapeutically effective amount of a peptide alone or in combination with: (a) an inhibitor of neutrophil Elastase or Mimosine; or (b) or Mimosine is administered in a pharmaceutical composition. In one embodiment, the peptides alone or the combinations with: (a) an inhibitor of neutrophil Elastase; or (b) or Mimosine described herein can be provided to the individual per-se. In one embodiment, the peptides described herein alone or in combination with: (a) an inhibitor of neutrophil Elastase; or (b) or Mimosine can be used as part of a pharmaceutical composition where it is mixed with a pharmaceutically acceptable carrier.

In another embodiment, treating a subject suffering from a disease characterized by tissue necrosis is inhibiting necrosis. In another embodiment, treating a subject suffering from a disease characterized by tissue necrosis is reducing the number of cells undergoing necrosis. In another embodiment, inhibiting necrosis is inhibiting necrosis in a cell exposed to pro-necrotic factors. In another embodiment, inhibiting necrosis is protecting a cell exposed to a necrotic inducing factor (such as but not limited to KCN) against necrosis. In another embodiment, inhibiting necrosis is inhibiting necrosis in a cell. In another embodiment, inhibiting necrosis is inhibiting necrosis in a tissue. In another embodiment, compositions described herein inhibit necrosis in a cell exposed to pro-necrotic factors. In another embodiment, compositions described herein inhibit necrosis in a cell, a tissue or an organ exposed to at least one pro-necrotic factor. In another embodiment, compositions described herein provide de-novo protection against necrosis in a cell, a tissue or an organ exposed to at least one pro-necrotic factor.

In another embodiment, use of a therapeutically effective amount of a peptide comprising an amino acid sequence selected from the group consisting of SEQ ID NOs:1-6 alone or in combination with a necrosis inhibitor, is for treating a subject suffering from a disease characterized by tissue necrosisIn another embodiment, the use of a therapeutically effective amount of a peptide consisting an amino acid sequence selected from the group consisting of SEQ ID NOs:1-6 alone or in combination with a necrosis inhibitor such as described herein is for treating a subject suffering from a disease characterized by tissue necrosis.

In another embodiment, use of the compositions disclosed herein is for inhibiting necrosis in cells, an organ or a tissue that caused a disease characterized by tissue necrosis. In another embodiment, use of the compositions disclosed herein is for protecting cells within an organ or a tissue affected with necrosis from necrosis. In another embodiment, use of the compositions disclosed herein is for reducing the number of necrotic cells with an organ or a tissue that caused the disease characterized by tissue necrosis.

In another embodiment, reducing the number of necrotic cells is reducing by at least 10%. In another embodiment, reducing the number of necrotic cells is reducing by at least 30%. In another embodiment, reducing the number of necrotic cells is reducing by at least 50%. In another embodiment, reducing the number of necrotic cells is reducing by at least 60%. In another embodiment, reducing the number of necrotic cells is reducing by at least 70%.

In another embodiment, use of a therapeutically effective amount of: (1) Humanin or a peptide comprising an amino acid sequence selected from the group consisting of SEQ ID NOs:1-6; and (2) a necrosis inhibitor such as described herein, inhibits tissue necrosis and the disease is characterized in that affected tissue in the subject is undergoing necrosis. In another embodiment, a necrosis inhibitor such as an inhibitor of neutrophil Elastase or Mimosineis used herein. In another embodiment, use includes any combination of peptides consisting an amino acid sequence selected from the group consisting of SEQ ID NOs:1-6.

In another embodiment, a necrosis inhibitor is an inhibitor of neutrophil Elastase. In another embodiment, inhibitor of neutrophil Elastase is Elastase inhibitor III. In another embodiment, Elastase inhibitor III is HLE Inhibitor MeOSuc-AAPV-CMK. In another embodiment, a necrosis inhibitor is Mimosine.

In another embodiment, use of a composition as described herein for inhibiting necrosis in a cell or a tissue comprises contacting the cell or tissue with a combination therapy of: (1) necrosis inhibitor; and (2) Humanin or a Humanin derivative according to SEQ ID NOs: 1-6. In another embodiment, a necrosis combination therapy as described herein (the combination of (1) necrosis inhibitor; and (2) Humanin or a Humanin derivative according to SEQ ID NOs: 1-6) comprises an unexpected synergistic anti-necrotic effect as provided in Example 2.

In another embodiment, the peptide comprising or consisting the amino acid sequence: MAPRGFSCLLLLTSEIDLPVKRRA (SEQ ID NO: 1). In another embodiment, the peptide comprising or consisting the amino acid sequence: MAPRGFSCLLLLTGEIDLPVKRRA (SEQ ID NO: 2). In another embodiment, the peptide comprising or consisting the amino acid sequence: MAPAGASCLLLLTGEIDLPVKRRA (SEQ ID NO: 3). In another embodiment, the peptide comprising or consisting the amino acid sequence: PRGFSCLLLLTSEIDLP (SEQ ID NO: 4). In another embodiment, the peptide comprising or consisting the amino acid sequence: PRGFSCLLLLTGEIDLP (SEQ ID NO: 5). In another embodiment, the peptide comprising or consisting the amino acid sequence: PAGASRLLLLTGEIDLP (SEQ ID NO: 6).

In another embodiment, the peptide described herein comprises or consists an amino acid sequence that is at least 50% homologous to the amino acid sequence of SEQ ID NOs: 1-6. In another embodiment, the peptide described herein comprises or consists an amino acid sequence that is at least 60% homologous to the amino acid sequence of SEQ ID NOs: 1-6. In another embodiment, the peptide described herein comprises or consists an amino acid sequence that is at least 70% homologous to the amino acid sequence of SEQ ID NOs: 1-6. In another embodiment, the peptide described herein comprises or consists an amino acid sequence that is at least 80% homologous to the amino acid sequence of SEQ ID NOs: 1-6. In another embodiment, the peptide described herein comprises or consists an amino acid sequence that is at least 85% homologous to the amino acid sequence of SEQ ID NOs: 1-6. In another embodiment, the peptide described herein comprises or consists an amino acid sequence that is at least 90% homologous to the amino acid sequence of SEQ ID NOs: 1-6. In another embodiment, the peptide described herein comprises or consists an amino acid sequence that is at least 95% homologous to the amino acid sequence of SEQ ID NOs: 1-6. In another embodiment, the peptide described herein comprises or consists an amino acid sequence that is at least 99% homologous to the amino acid sequence of SEQ ID NOs: 1-6.

In another embodiment, the peptide described herein comprises or consists an amino acid sequence that is at least 50% identical to the amino acid sequence of SEQ ID NOs: 1-6. In another embodiment, the peptide described herein comprises or consists an amino acid sequence that is at least 60% identical to the amino acid sequence of SEQ ID NOs: 1-6. In another embodiment, the peptide described herein comprises or consists an amino acid sequence that is at least 70% identical to the amino acid sequence of SEQ ID NOs: 1-6. In another embodiment, the peptide described herein comprises or consists an amino acid sequence that is at least 80% identical to the amino acid sequence of SEQ ID NOs: 1-6. In another embodiment, the peptide described herein comprises or consists an amino acid sequence that is at least 85% identical to the amino acid sequence of SEQ ID NOs: 1-6. In another embodiment, the peptide described herein comprises or consists an amino acid sequence that is at least 90% identical to the amino acid sequence of SEQ ID NOs: 1-6. In another embodiment, the peptide described herein comprises or consists an amino acid sequence that is at least 95% identical to the amino acid sequence of SEQ ID NOs: 1-6. In another embodiment, the peptide described herein comprises or consists an amino acid sequence that is at least 99% identical to the amino acid sequence of SEQ ID NOs: 1-6.

In another embodiment, the peptide described herein is a fusion protein of an above-mentioned peptide with other peptides/polypeptides. In another embodiment, a fusion protein is a polypeptide in which at least two peptides that are not bound in nature are joined, and can be produced by peptide synthesis, or by expressing nucleic acids wherein the peptide encoding regions are ligated in frame. Examples of other polypeptides that are fused to the protein described herein include arbitrary polypeptides comprising short peptides with few residues, such as tags, and long polypeptides, such as proteins. Specifically, such examples include His tag, HA tag, GFP, maltose binding protein, and glutathione S-transferase (GST). Additionally, antibody fragments (Fc fragment), and such may be also used. Other examples include leader sequence, secretion signal, and preprotein or proprotein sequences, but the present disclosure is not limited to these examples. Further, a group of polypeptides, that facilitates the peptide described herein to effectively pass the blood-brain barrier, can be fused to the protein described herein.

In another embodiment, the peptide described herein includes derivatives of the peptides described hereinabove. In another embodiment, the term "derivatives" refers to molecules that have a form, which has been altered by modification, addition, mutation, substitution, or deletion of functional groups of the peptide described herein according to conventional methods. Such alterations of functional groups are carried out, for example, to protect functional groups of the peptide, to regulate the stability or histological localization of the peptide, or to regulate the activity of the peptide, and so on. In another embodiment, the peptides described herein are exemplified by those peptides wherein any one of the N-terminus, C-terminus, and functional groups of the peptides constituting amino acid side chains are modified by substituents, such as protecting groups. In another embodiment, the substituents include, for example, various alkyl groups, acyl groups, amide groups, phosphate groups, amino groups, carboxyl groups, and ester groups; however, the present disclosure is not limited to these examples.

In another embodiment, the peptides described herein are bound to polymers, such as dimers wherein the peptides are bound to each other; branched molecules; and cyclized molecules. In another embodiment, the peptide may be bound to a carrier. For example, the peptide described herein may be bound to polyethylene glycol (PEG), dextran, other polymers, and so on.

In another embodiment, amino acids that constitute the peptides described herein are in the L form and/or D form. In another embodiment, D amino acids are effective for lowering degradation by peptidases. In another embodiment, the amino acids are not limited to natural amino acids, and may be also unnatural amino acids. In another embodiment, unnatural amino acids are exemplified by homoserine, beta-hydroxyvaline, 0-4-hydroxyphenyl tyrosine, alpha-t-butyl glycine, 2-amine butyrate, alpha-cyclohexyl glycine, alpha-phenyl glycine, and such.

In another embodiment, the peptide bonds of a peptide as described herein are substituted with covalent bonds other than peptide bonds. In another embodiment, sensitivity to proteases/peptidases of the peptides can be lowered by the substitution to non-peptide bonds, which enhances drug efficacy duration and which offers a wide selection of administration routes. In another embodiment, a non-peptide bond is exemplified by imino bonds, ester bonds, hydrazine bonds, semicarbazide bonds, and azo bonds, but the present disclosure is not limited to these examples.

In another embodiment, further provided herein chemical compounds, that mimic the structure of the peptides as described herein. In another embodiment, based on the physical and chemical properties (which may be analyzed by conventional methods including active site modification, NMR, and X-ray crystallography) relating to the structure of the peptides described herein a map of physical and chemical functions, that are important for protective action of the peptides, is constructed.

In another embodiment, compositions described herein comprising a peptide as described herein are used to ameliorate, reverse, and/or treat diseases and/or symptoms associated with necrosis. In another embodiment, compositions described herein comprising an effective amount of: (1) Humanin or a peptide comprising an amino acid sequence selected from the group consisting of SEQ ID NOs:1-6; and (2) a necrosis inhibitor, are used to ameliorate, reverse, and/or treat diseases and/or symptoms associated with necrosis

In some embodiments, modifications of a peptide described herein include, but are not limited to N-terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are described in Quantitative Drug Design, C.A. Ramsden Gd., Chapter 17.2, F. Choplin Pergamon Press (1992). Further details in this respect are provided hereinunder.

In some embodiments, peptide bonds (-CO-NH-) within a peptide described herein are substituted. In some embodiments, peptide bonds are substituted by N-methylated bonds (-N(CH3)-CO-). In some embodiments, the peptide bonds are substituted by ester bonds (-C(R)H-C-O-O-C(R)-N-). In some embodiments, the peptide bonds are substituted by ketomethylen bonds (-CO-CH2-). In some embodiments, the peptide bonds are substituted by -aza bonds (-NH-N(R)-CO-), wherein R is any alkyl, e.g., methyl, carba bonds (-CH2-NH-). In some embodiments, the peptide bonds are substituted by hydroxyethylene bonds (-CH(OH)-CH2-). In some embodiments, the peptide bonds are substituted by thioamide bonds (-CS-NH-). In some embodiments, the peptide bonds are substituted by olefinic double bonds (-CH=CH-). In some embodiments, the peptide bonds are substituted by retro amide bonds (-NH-CO-). In some embodiments, the peptide bonds are substituted by peptide derivatives (-N(R)-CH2-CO-), wherein R is the "normal" side chain, naturally presented on the carbon atom. In some embodiments, these modifications occur at any of the bonds along the peptide chain and even at several (2-3 bonds) at the same time.

In some embodiments, natural aromatic amino acids of a peptide such as Trp, Tyr and Phe, are substituted for synthetic non-natural acid such as Phenylglycine, TIC, naphthylelanine (Nol), ring-methylated derivatives of Phe, halogenated derivatives of Phe or o-methyl-Tyr. In some embodiments, the peptide described herein includes one or more modified amino acid or one or more non-amino acid monomers (e.g. fatty acid, complex carbohydrates etc).

In one embodiment, "amino acid" is understood to include the 20 naturally occurring amino acid; those amino acid often modified post-translationally in vivo, including, for example, hydroxyproline, phosphoserine and phosphothreonine; and other unusual amino acid including, but not limited to, 2-aminoadipic acid, hydroxylysine, isodesmosine, nor-valine, nor-leucine and ornithine. In one embodiment, "amino acid" includes both D- and L-amino acid.

In other embodiments, a disease is characterized in that at least 51 % of affected tissue in the subject is undergoing necrosis as opposed to apoptosis. Further described herein are prophylactic usages of a therapeutically effective amount of a peptide described herein such that the effective amount inhibits tissue necrosis in a subject for a subject at risk for a pathological condition that is precipitated at least in part by tissue necrosis.

Further disclosed is a prophylactic usage of (1) Humanin or a peptide comprising an amino acid sequence selected from the group consisting of SEQ ID NOs:1-6; and (2) a necrosis inhibitor for a subject at risk for a pathological condition that is precipitated at least in part by tissue necrosis..

In another embodiment, inhibition of tissue necrosis is the reduction in number of necrotic cells. In another embodiment, inhibition of tissue necrosis is the inhibition of de-novo necrosis achievable by the administration of a peptide as described herein. In another embodiment, disclosed herein is the use of a peptide of described herein for inhibiting necrosis in a cell or tissue culture.

In another embodiment, necrosis is one of the pathologies seen in several diseases. For example, a disease characterized by tissue necrosis, is diabetes or open wounds which are not treated may result in the development of necrosis. In another embodiment, a disease characterized by tissue necrosis comprises cells or a tissue that do not receive oxygen for a prolonged period of time. This is evident in cardiac infarction and in stroke, where the related tissue is demonstrably affected .In another embodiment, a prophylactic treatment such as described herein is suitable to disease wherein necrosis is one of the known pathologies.

In another embodiment, necrosis comprises aseptic necrosis which is bone death caused by poor blood supply to the area. In another embodiment, aseptic necrosis is common in the hip, knee, and shoulder. Aseptic necrosis occurs when at least part of a bone is poorly perfused. Under such circumstances, part(s) of the bone fractures. If this condition is not treated, bone damage worsens, and remaining healthy/unaffected regions of the bone may collapse. In another embodiment, aseptic necrosis is treated by the usages described herein.

In another embodiment, necrosis arises from dead tissue formation at a site of radiation-radiation necrosis, which forms from radiation cancer therapy. In another embodiment, radiation necrosis is treated by the usages described herein. In some aspects, the mass of dead tissue contains both cancerous and healthy cells. Radiation necrosis can develop over a period of months to years, providing a reasonable venue for prophylactic treatment such as described herein of such patients. In another embodiment, radiation necrosis results in dementia, headache and seizures. In another embodiment, the usages described herein prevent dementia, headache and seizures resulting from radiation necrosis.

In another embodiment, the composition described herein is: a peptide comprising or consisting an amino acid sequence selected from the group consisting of SEQ ID NOs:1-6. In another embodiment, the composition described herein is Humanin or a peptide comprising an amino acid sequence selected from the group consisting of SEQ ID NOs:1-6; and (2) a necrosis inhibitor. In another embodiment, the phrases: the composition described herein, compositions and composition are used interchangeably.

In another embodiment, the composition described herein is effective in inhibiting necrosis. In another embodiment, the compositions described herein are effective in protecting a cell or a tissue exposed to pro-necrotic factors. In another embodiment, composition as described herein is effective in maintaining the viability of cells or a tissue exposed to pro-necrotic factors. In another embodiment, composition as described herein prevents the devastating necrotic effects exerted by pro-necrotic factors. In another embodiment, composition as described herein prevents or delays necrosis. In another embodiment, composition as described herein is used as preventive measure against necrosis. In another embodiment, composition as described herein counteract the pro-necrotic effect of factors that induce necrosis. In another embodiment, composition as described herein reduces the number of necrotic cell in a tissue exposed to pro-necrotic factors. In another embodiment, composition as described herein protects cells from necrosis. In another embodiment, the cells are susceptible or exposed to pro-necrotic factors.

In one embodiment, provided herein a usage for increasing cell viability in a necrotic tissue. In one embodiment, provided herein a usage for increasing cell viability in a pre-necrotic tissue. In one embodiment, provided herein a usage for protecting a cell against necrosis induced by a necrosis inducing agent. In another embodiment, a necrosis inducing agent is an endogenic factor or an exogenic factor. In another embodiment, the composition described herein can rescue a necrotic cell. In another embodiment, the composition described herein can rescue a necrotic cell regardless of the underlying cause of necrosis.

In another embodiment, necrosis arises from soft-tissue infection which is a severe type of tissue infection that can involve the skin, subcutaneous fat, the muscle sheath (fascia), and the muscle. In another embodiment, necrosis causes gangrene, tissue death, systemic disease and death. In another embodiment, a peptide as described herein inhibits or protects against necrosis in a necrotizing subcutaneous infection or fasciitis. In another embodiment, necrotizing subcutaneous infection or fasciitis is treated by a peptide as described herein combined with an antibiotic.

In another embodiment, the composition described herein inhibits or protects against necrosis in a soft tissue. In another embodiment, the usage described herein prevent the need of a surgery required to open and drain infected areas and remove dead tissue. In another embodiment, a use of a composition as described herein for reducing the symptoms associated with a disease such as described herein (necrotic disease) in a subject is provided. In another embodiment, use of a composition as described herein for curing a necrotic disease in a subject, is provided. In another embodiment, use of a composition as described herein for ameliorating a necrotic disease in a subject, is provided. In another embodiment, use of a composition as described herein for improving the wellbeing of a subject afflicted with a necrotic disease, is provided.

In another embodiment, provided herein is a use of a composition as described herein for preventing pancreatitis in a subject at risk of being afflicted with pancreatitis. In another embodiment, provided herein is a use of a composition as described herein for reducing the severity of pancreatitis in a subject. In another embodiment, provided herein is a use of a composition as described herein for reducing the symptoms associated with of pancreatitis in a subject. In another embodiment, provided herein is a use of a composition as described herein for treating pancreatitis in a subject. In another embodiment, use of a composition as described herein for curing pancreatitis. In another embodiment, use of a composition as described herein for ameliorating pancreatitis in a subject afflicted with pancreatitisis described herein. In another embodiment, provided herein is a use of a composition as described herein for improving the wellbeing of a subject afflicted with pancreatitis.

In another embodiment, preventing comprises reducing the risk of pancreatitis. In another embodiment, preventing pancreatitis comprises reducing the severity of pancreatitis.

In another embodiment, use of a composition in combination with an additional active pharmaceutical ingredient prior to an abdominal surgical procedure as described herein is for preventing pancreatitis.

In another embodiment, pancreatitis is induced by a pancreatitis causing medicine. In another embodiment, a pancreatitis causing medicine is an AIDS drug. In another embodiment, a pancreatitis causing medicine is a DDI. In another embodiment, a pancreatitis causing medicine is pentamidine. In another embodiment, a pancreatitis causing medicine is a diuretic. In another embodiment, a pancreatitis causing medicine is furosemide. In another embodiment, a pancreatitis causing medicine is hydrochlorothiazide. In another embodiment, a pancreatitis causing medicine is an anticonvulsant. In another embodiment, a pancreatitis causing medicine is divalproex sodium. In another embodiment, a pancreatitis causing medicine is valproic acid. In another embodiment, a pancreatitis causing medicine is L-asparaginase. In another embodiment, a pancreatitis causing medicine is azathioprine. In another embodiment, a pancreatitis causing medicine is estrogen.

In another embodiment, use is for of preventing iatrogenic procedure-related acute pancreatitis. In another embodiment, use is for preventing pancreatitis caused by any pancreatic surgical procedure known to one of skill in the art. In another embodiment, use is for preventing pancreatitis by inhibiting necrosis.

In another embodiment, an effective amount of a peptide as described herein is between about 0.2 to 500 mg/kg/day of body weight. In another embodiment, an effective amount of a peptide as described herein is between about 20 to 500 mg/kg/day of body weight. In another embodiment, an effective amount of a peptide as described herein is between about 30 to 250 mg/kg/day of body weight. In another embodiment, an effective amount of a peptide as described herein is between about 50 to 150 mg/kg/day of body weight.

In another embodiment, an effective amount of a necrosis inhibitor is between about 0.2 to 500 mg/kg/day of body weight. In another embodiment, an effective amount of a necrosis inhibitor is between about 20 to 500 mg/kg/day of body weight. In another embodiment, an effective amount of a necrosis inhibitor is between about 30 to 250 mg/kg/day of body weight. In another embodiment, an effective amount of a necrosis inhibitor is between about 50 to 150 mg/kg/day of body weight.

In some embodiments, use of the compositions described herein is followed by analysis of the necrotic process and determination whether the necrotic process is inhibited by the treatment with the compositions described herein. This may be conducted, in some embodiments, by taking a biopsy from the site of necrosis and analysis of the biopsy with the common distinctive procedures for detection of necrosis. These assays include, but are not limited, in some embodiments, to differential staining such as the combined stain of acridine orange and ethydium bromide. Acridine orange (AO) permeates all cells and makes the nuclei appear green. Ethidium bromide (EB) is only taken up by cells when cytoplasmic membrane integrity is lost, and stains the nucleus red. EB also dominates over AO. Thus live cells have a normal green nucleus; early apoptotic cells have bright green nucleus with condensed or fragmented chromatin; late apoptotic cells display condensed and fragmented orange chromatin; cells that have died from direct necrosis have a structurally normal orange nucleus. In another embodiment, a method for measuring cytotoxicity in cells such as lactate dehydrogenase (LDH) release from dying necrotic cells can indicate necrosis. Lactate dehydrogenase is a cytosolic enzyme present within all mammalian cells. The normal plasma membrane is impermeable to LDH, but damage to the cell membrane results in a change in the membrane permeability and subsequent leakage of LDH into the extracellular fluid. In-Vitro release of LDH from cells provides an accurate measure of cell membrane integrity and cell viability. This assay is based upon the ability of LDH to catalyze the reaction: Lactate(-) + NAD(+) --> Pyruvate + NADH. Changes in optical absorbance, measured at 340nm, reflect changes in the concentration of NADH and hence the level of LDH in the test sample.

In some embodiments, cell viability assays such as trypan blue staining are used to assess cellular necrosis. Since cells are highly selective in the compounds that pass through the membrane, in a viable cell trypan blue is not absorbed, however, it traverses the membrane in a dead cell. Hence, dead cells exhibit a distinctive blue color under a microscope. In some embodiments, use of the compositions described herein as described herein is followed by monitoring the availability of the peptide at the necrotic tissue by taking a biopsy from the necrotic area and immunoassaying for the presence of the in the sample. In another embodiment, monitoring the use of compositions as described herein may be accomplished by imaging of the peptide distribution at the site of necrosis. This can be done by linking a peptide as described herein to a specific marker which enables tracking and detection using an imaging device. In some embodiments, the usage of PET scan can revel the existence of a necrotic tissue and asses the efficacy of treatment with the compositions.

In some embodiments, treatment of necrosis may require additional medicaments to be administered in parallel to the compositions as described herein. For example, in one embodiment, treatment of diabetes complications resulting in diabetic necrotic wounds may consist, in parallel to the present treatment, antibiotics, anti-inflammatory drugs and insulin.

In some embodiments, provided herein a use of a composition for treating necrosis in a subject refractory to anti-inflammatory drugs. In another embodiment, the necrotic disease is a result of severe inflammation leading to the development of necrotic tissue. In another embodiment, a subject is non responsive to such anti-inflammatory treatment, specific treatment of the necrosis such as described herein is a viable alternative solution.

In some embodiments, a disease treatable or reversible by the uses described herein is cancer, neurodegenerative disease, myocardial infarction, stroke, sepsis, ischemia, liver disease, open wounds, organ transplants or gangrene. In some embodiments, the patient is immunocompromised. In one embodiment, a patient suffering from AIDS dementia, a necrotic process in brain cells specifically macrophages and microglia, may benefit from treatment with the compositions as described herein. Brain cells infected with HIV, secrete neurotoxins of both host and viral origin resulting in death of brain cells. The essential features of AIDS dementia are disabling cognitive impairment accompanied by motor dysfunction, speech problems and behavioral change. In one embodiment, treatment with the compositions described herein reduces the necrotic cell death which leads to the devastating development of AIDS dementia.

In another embodiment, provided herein a prophylactic use of a subject at risk for a pathological condition that is precipitated at least in part by tissue necrosis. In another embodiment, such conditions are, but not limited to, diabetes, cancer, neurodegenerative disease, myocardial infarction, stroke, sepsis, ischemia, liver disease and transplant patients. In another embodiment, a patient is pre-treat with the composition described herein to avoid the development of necrosis during the progress of the disease or due to the treatment of the disease, in the absence of effective therapy (transplantation). In some embodiments, prophylactic treatment includes administering a subject a therapeutically effective amount of the compositions described herein to effectively inhibit the potential development of necrosis. In some embodiments, the compositions described herein inhibit a venom induction of rapid necrosis. In some embodiments, the compositions described herein rescues tissue susceptible to necrosis induced by venom. In another embodiment, administration of the composition described herein to a victim of a poisonous bite results in inhibition of the necrotic process. This may be done, in some embodiments, by injection or by topical application of the compositions as described herein.

In some embodiments, the compositions as described herein are useful in the treatment of any disease characterized by necrosis. In some embodiments, such diseases may comprise neurodegenerative disorders, leukemias, lymphomas, neonatal respiratory distress, asphyxia, incarcerated hernia, diabetes, tuberculosis, endometriosis, vascular dystrophy, psoriasis, cold injury, iron-load complications, complications of steroid treatment, ischemic heart disease, reperfusion injury, cerebrovascular disease or damage, gangrene, pressure sores, pancreatitis, hepatitis, hemoglobinuria, bacterial sepsis, viral sepsis, burns, hyperthermia, Crohn's disease, celiac disease, compartment syndrome, necrotizing procolitis, cystic fibrosis, rheumatoid arthritis, nephrotoxicity, multiple sclerosis, spinal cord injury, glomerulonephritis, muscular dystrophy, degenerative arthritis, tyromesia, metabolic inherited disease, mycoplasmal disease, anthrax infection, bacterial infection, viral infection, Anderson disease, congenital mitochondrial disease, phenylketonuria, placental infarct, syphilis, asceptic necrosis, avascular necrosis, alcoholism and necrosis associated with administration and/or self-administration with, and/or exposure to, cocaine, drugs (e.g. paracetamol, antibiotics, adriamycin, NSAID, cyclosporine) chemical toxins such as carbon tetrachloride, cyanide, methanol, ethylene glycol and mustard gas, agrochemicals such as organophosphates and aging.

In another embodiment necrosis is induced in cells or tissue culture due to lack of oxygen, inhibition of biochemical respiratory cycle, or various toxins. Necrosis in cells or tissue culture due to lack of oxygen, inhibition of biochemical respiratory cycle, or various toxins may result in loss of the culture and the valuable time and effort invested in establishing this culture. In one embodiment, treating a cell culture with the compositions inhibit necrosis may lead to prevention of the loss of the culture. In another embodiment, a culture prone to necrotic cell death might serve as an experimental system for the study of necrosis. In one embodiment, supplying to such culture sufficient amount of the compositions to inhibit the necrotic death and subsequent removal of the compositions when assaying for the process of necrosis may result in an efficient inducible cell system for the study of necrosis. In some embodiments, the compositions described herein are used to prevent necrosis in sustaining tissues and whole organs before transplantation. In one embodiment, a tissue whether a part of or a whole organ is treated with a composition as described herein to inhibit necrosis and sustain the initial condition of the organ, or in some embodiments, allow for prolonged organ culture.

In some embodiments, the peptide described herein is chemically synthesized such as by using standard solid phase techniques. In some embodiments, these chemical methods include exclusive solid phase synthesis, partial solid phase synthesis, fragment condensation, or classical solution synthesis.

In some embodiments, solid phase peptide synthesis procedures are well known to one skilled in the art and further described by John Morrow Stewart and Janis Dillaha Young, Solid Phase peptide Syntheses (2nd Ed., Pierce Chemical Company, 1984). In some embodiments, synthetic peptide is purified by preparative high performance liquid chromatography [Creighton T. (1983) Proteins, structures and molecular principles. WH Freeman and Co. N.Y.] and the composition of which can be confirmed via amino acid analysis and mass spectra analysis by methods known to one skilled in the art.

In some embodiments, recombinant protein techniques are used to generate the peptide described herein. In some embodiments, recombinant protein techniques are used for generation of a peptide (e.g., longer than 18-25 amino acids). In some embodiments, recombinant protein techniques are used for the generation of large amounts of the peptide described herein. In some embodiments, recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463 .

In one embodiment, a peptide described herein is synthesized using a polynucleotide encoding a peptide described herein. In some embodiments, the polynucleotide encoding a peptide described herein is ligated into an expression vector, comprising a transcriptional control of a cis-regulatory sequence (e.g., promoter sequence). In some embodiments, the cis-regulatory sequence is suitable for directing constitutive expression of the peptide described herein.

In some embodiments, the cis-regulatory sequence is suitable for directing tissue specific expression of the peptide described herein. In some embodiments, the cis-regulatory sequence is suitable for directing inducible expression of the peptide described herein.

In some embodiment, tissue-specific promoters suitable for use with the present sequences which are functional in specific cell population, example include, but are not limited to promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166). Inducible promoters suitable for use include for example the tetracycline-inducible promoter (Srour, M.A., et al., 2003. Thromb. Haemost. 90: 398-405).

In one embodiment, the phrase "a polynucleotide" refers to a single or double stranded nucleic acid sequence which be isolated and provided in the form of an RNA sequence, a complementary polynucleotide sequence (cDNA), a genomic polynucleotide sequence and/or a composite polynucleotide sequences (e.g., a combination of the above).

In one embodiment, "complementary polynucleotide sequence" refers to a sequence, which results from reverse transcription of messenger RNA using a reverse transcriptase or any other RNA dependent DNA polymerase. In one embodiment, the sequence can be subsequently amplified in vivo or in vitro using a DNA polymerase.

In another embodiment, "genomic polynucleotide sequence" refers to a sequence derived (isolated) from a chromosome and thus it represents a contiguous portion of a chromosome.

In one embodiment, "composite polynucleotide sequence" refers to a sequence, which is at least partially complementary and at least partially genomic. In one embodiment, a composite sequence can include some exonal sequences required to encode the peptide described herein, as well as some intronic sequences interposing therebetween. In one embodiment, the intronic sequences can be of any source, including of other genes, and typically will include conserved splicing signal sequences. In one embodiment, intronic sequences include cis acting expression regulatory elements.

In some embodiments, polynucleotides described herein are prepared using PCR techniques, or any other method or procedure known to one skilled in the art. In some embodiments, the procedure involves the ligation of two different DNA sequences (See, for example, "Current Protocols in Molecular Biology", eds. Ausubel et al., John Wiley & Sons, 1992).

In one embodiment, polynucleotides described herein are inserted into expression vectors (i.e., a nucleic acid construct) to enable expression of the recombinant polypeptide. In one embodiment, the expression vector described herein includes additional sequences which render this vector suitable for replication and integration in prokaryotes. In one embodiment, the expression vector described herein includes additional sequences which render this vector suitable for replication and integration in eukaryotes. In one embodiment, the expression vector described herein includes a shuttle vector which renders this vector suitable for replication and integration in both prokaryotes and eukaryotes. In some embodiments, cloning vectors comprise transcription and translation initiation sequences (e.g., promoters, enhances) and transcription and translation terminators (e.g., polyadenylation signals).

In one embodiment, a variety of prokaryotic or eukaryotic cells can be used as host-expression systems to express the peptides described herein. In some embodiments, these include, but are not limited to, microorganisms, such as bacteria transformed with a recombinant bacteriophage DNA, plasmid DNA or cosmid DNA expression vector containing the peptide coding sequence; yeast transformed with recombinant yeast expression vectors containing the peptide coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the peptide coding sequence.

In some embodiments, non-bacterial expression systems are used (e.g. mammalian expression systems such as CHO cells) to express the peptide described herein. In one embodiment, the expression vector used to express polynucleotides described herein in mammalian cells is pCI-DHFR vector comprising a CMV promoter and a neomycin resistance gene.

In some embodiments, in bacterial systems described herein, a number of expression vectors can be advantageously selected depending upon the use intended for the peptide expressed. In one embodiment, large quantities of the peptide are desired. In one embodiment, vectors that direct the expression of high levels of the peptide product, possibly as a fusion with a hydrophobic signal sequence, which directs the expressed product into the periplasm of the bacteria or the culture medium where the protein product is readily purified are desired. In one embodiment, certain fusion protein engineered with a specific cleavage site to aid in recovery of the peptide. In one embodiment, vectors adaptable to such manipulation include, but are not limited to, the pET series of E. coli expression vectors [Studier et al., Methods in Enzymol. 185:60-89 (1990)].

In one embodiment, yeast expression systems are used. In one embodiment, a number of vectors containing constitutive or inducible promoters can be used in yeast as disclosed in U.S. Pat. Application No: 5,932,447. In another embodiment, vectors which promote integration of foreign DNA sequences into the yeast chromosome are used.

In one embodiment, the expression vector described herein can further include additional polynucleotide sequences that allow, for example, the translation of several proteins from a single mRNA such as an internal ribosome entry site (IRES) and sequences for genomic integration of the promoter-chimeric peptide.

In some embodiments, mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

In some embodiments, expression vectors containing regulatory elements from eukaryotic viruses such as retroviruses are used. SV40 vectors include pSVT7 and pMT2. In some embodiments, vectors derived from bovine papilloma virus include pBV-1MTHA, and vectors derived from Epstein Bar virus include pHEBO, and p2O5. Other exemplary vectors include pMSG, pAV009/A+, pMTO10/A+, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

In some embodiments, recombinant viral vectors are useful for in vivo expression of the peptide described herein since they offer advantages such as lateral infection and targeting specificity. In one embodiment, lateral infection is inherent in the life cycle of, for example, retrovirus and is the process by which a single infected cell produces many progeny virions that bud off and infect neighboring cells. In one embodiment, the result is that a large area becomes rapidly infected, most of which was not initially infected by the original viral particles. In one embodiment, viral vectors are produced that are unable to spread laterally. In one embodiment, this characteristic can be useful if the desired purpose is to introduce a specified gene into only a localized number of targeted cells

In one embodiment, various methods can be used to introduce the expression vector described herein into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et al. [Biotechniques 4 (6): 504-512, 1986] and include, for example, stable or transient transfection, lipofection, electroporation and infection with recombinant viral vectors. In addition, see U.S. Patent Nos. 5,464,764 and 5,487,992 for positive-negative selection methods.

In one embodiment, plant expression vectors are used. In one embodiment, the expression of the peptide coding sequence is driven by a number of promoters. In some embodiments, viral promoters such as the 35S RNA and 19S RNA promoters of CaMV [Brisson et al., Nature 310:511-514 (1984)], or the coat protein promoter to TMV [Takamatsu et al., EMBO J. 6:307-311 (1987)] are used. In another embodiment, plant promoters are used such as, for example, the small subunit of RUBISCO [Coruzzi et al., EMBO J. 3:1671-1680 (1984); and Brogli et al., Science 224:838-843 (1984)] or heat shock promoters, e.g., soybean hsp17.5-E or hsp17.3-B [Gurley et al., Mol. Cell. Biol. 6:559-565 (1986)]. In one embodiment, constructs are introduced into plant cells using Ti plasmid, Ri plasmid, plant viral vectors, direct DNA transformation, microinjection, electroporation and other techniques well known to the skilled artisan. See, for example, Weissbach & Weissbach [Methods for Plant Molecular Biology, Academic Press, NY, Section VIII, pp 421-463 (1988)]. Other expression systems such as insects and mammalian host cell systems, which are well known in the art, can also be used.

It will be appreciated that other than containing the necessary elements for the transcription and translation of the inserted coding sequence (encoding the peptide or protein), the expression construct described herein can also include sequences engineered to optimize stability, production, purification, yield or activity of the expressed peptide or protein.

In some embodiments, transformed cells are cultured under effective conditions, which allow for the expression of high amounts of recombinant peptide. In some embodiments, effective culture conditions include, but are not limited to, effective media, bioreactor, temperature, pH and oxygen conditions that permit protein production. In one embodiment, an effective medium refers to any medium in which a cell is cultured to produce the recombinant peptide or protein described herein. In some embodiments, a medium typically includes an aqueous solution having assimilable carbon, nitrogen and phosphate sources, and appropriate salts, minerals, metals and other nutrients, such as vitamins. In some embodiments, cells described herein can be cultured in conventional fermentation bioreactors, shake flasks, test tubes, microtiter dishes and petri plates. In some embodiments, culturing is carried out at a temperature, pH and oxygen content appropriate for a recombinant cell. In some embodiments, culturing conditions are within the expertise of one of ordinary skill in the art.

In some embodiments, depending on the vector and host system used for production, resultant peptides or proteins described herein either remain within the recombinant cell, secreted into the fermentation medium, secreted into a space between two cellular membranes, or retained on the outer surface of a cell or viral membrane.

In one embodiment, following a predetermined time in culture, recovery of the recombinant peptide or protein is effected.

In one embodiment, the phrase "recovering the recombinant peptide or protein" used herein refers to collecting the whole fermentation medium containing the peptide or protein and need not imply additional steps of separation or purification.

In one embodiment, peptides or proteins described herein are purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

In one embodiment, to facilitate recovery, the expressed coding sequence can be engineered to encode the peptide or proteins described herein and fused cleavable moiety. In one embodiment, a fusion protein can be designed so that the peptide or protein can be readily isolated by affinity chromatography; e.g., by immobilization on a column specific for the cleavable moiety. In one embodiment, a cleavage site is engineered between the peptide or protein and the cleavable moiety and the peptide or protein can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)].

In one embodiment, the peptide or protein described herein is retrieved in "substantially pure" form.

In one embodiment, the phrase "substantially pure" refers to a purity that allows for the effective use of the protein in the applications described herein.

In one embodiment, the peptide or protein described herein can also be synthesized using *in vitro* expression systems. In one embodiment, *in vitro* synthesis methods are well known in the art and the components of the system are commercially available.

In some embodiments, the proteins or peptides described herein can be modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. In another embodiment, the modified proteins or peptides described herein exhibit substantially longer half-lives in blood following intravenous injection than do the corresponding unmodified compounds. In one embodiment, modifications also increase the proteins or peptides solubility in aqueous solution, eliminate aggregation, enhance the physical and chemical stability of the compound, and greatly reduce the immunogenicity and reactivity of the compound. In another embodiment, the desired *in vivo* biological activity is achieved by the administration of such polymer-compound abducts less frequently or in lower doses than with the unmodified compound.

In one embodiment, a "pharmaceutical composition" refers to a preparation of one or more of the active ingredients described herein with other chemical components such as physiologically suitable carriers and excipients. The purpose of a pharmaceutical composition is to facilitate administration of a peptide to an organism.

In one embodiment, "active ingredient" refers to the peptide, Humanin, and/or necrosis inhibitor alone or in combination as described herein, which is accountable for the biological effect. In one embodiment, "active ingredient" refers to the peptide, Humanin, and/or necrosis inhibitor as described herein, which is accountable for the anti-necrotic effect.

In one embodiment, the phrases "physiologically acceptable carrier" and "pharmaceutically acceptable carrier" which be interchangeably used refer to a carrier or a diluent that does not cause significant irritation to an organism and does not abrogate the biological activity and properties of the administered compound. An adjuvant is included under these phrases. In one embodiment, one of the ingredients included in the pharmaceutically acceptable carrier can be for example polyethylene glycol (PEG), a biocompatible polymer with a wide range of solubility in both organic and aqueous media (Mutter et al. (1979).

In one embodiment, "excipient" refers to an inert substance added to a pharmaceutical composition to further facilitate administration of an active ingredient. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

In one embodiment, suitable routes of administration, for example, include oral, rectal, transmucosal, transnasal, intestinal or parenteral delivery, including intramuscular, subcutaneous and intramedullary injections as well as intrathecal, direct intraventricular, intravenous, inrtaperitoneal, intranasal, or intraocular injections.

In some embodiments, compositions for use as described herein comprise solutions or emulsions, which in some embodiments are aqueous solutions or emulsions comprising a safe and effective amount of the compounds described herein and optionally, other compounds, intended for topical intranasal administration. In some embodiments, these compositions comprise from about 0.01% to about 10.0% w/v of the peptide described herein, or from about 0.1% to about 2.0.

In another embodiment, the pharmaceutical compositions are administered by intravenous, intra-arterial, or intramuscular injection of a liquid preparation. In some embodiments, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the compounds described herein are combined with an additional appropriate therapeutic agent or agents, prepared and applied as solutions, suspensions, or emulsions in a physiologically acceptable diluent with or without a pharmaceutical carrier.

In one embodiment, pharmaceutical compositions for use are formulated in conventional manner using one or more physiologically acceptable carriers comprising excipients and auxiliaries, which facilitate processing of the active ingredients into preparations which, can be used pharmaceutically. In one embodiment, formulation is dependent upon the route of administration chosen.

In one embodiment, injectables, are formulated in aqueous solutions. In one embodiment, injectables, are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In some embodiments, formulations for injection are presented in unit dosage form, e.g., in ampoules or in multidose containers with optionally, an added preservative. In some embodiments, compositions are suspensions, solutions or emulsions in oily or aqueous vehicles, and contain formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjusters, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In some embodiments, pharmaceutical compositions for parenteral administration include aqueous solutions of the active preparation in water-soluble form. Additionally, suspensions of the active ingredients, in some embodiments, are prepared as appropriate oily or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances, which increase the viscosity of the suspension, such as sodium carboxymethyl cellulose, sorbitol or dextran. In another embodiment,, the suspension also contain suitable stabilizers or agents which increase the solubility of the active ingredients to allow for the preparation of highly concentrated solutions.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid.

In another embodiment, the pharmaceutical composition delivered in a controlled release system is formulated for intravenous infusion, implantable osmotic pump, transdermal patch, liposomes, or other modes of administration. In one embodiment, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In some embodiments, pharmaceutical compositions suitable for use in context described herein include compositions wherein peptide, Humanin, and/or necrosis inhibitor as described herein is/are contained in an amount effective to achieve protection against necrosis or inhibition of necrosis. In some embodiments, a therapeutically effective amount means an amount of a peptide, Humanin, and/or necrosis inhibitor effective to prevent, alleviate or ameliorate symptoms of disease associated with necrosis or prolong the survival of the subject being treated. In one embodiment, determination of a therapeutically effective amount is well within the capability of those skilled in the art.

In some embodiments, preparation of effective amount or dose can be estimated initially from in vitro assays. In one embodiment, a dose can be formulated in animal models and such information can be used to more accurately determine useful doses in humans.

In one embodiment, toxicity and therapeutic efficacy of the active ingredients described herein can be determined by standard pharmaceutical procedures in vitro, in cell cultures or experimental animals. In one embodiment, the data obtained from these in vitro and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975) "The Pharmacological Basis of Therapeutics", Ch. 1 p.1].

In one embodiment, depending on the severity and responsiveness of the condition to be treated, dosing can be of a single or a plurality of administrations, with course of treatment lasting from several days to several weeks or until cure is effected or diminution of the disease state is achieved.

In one embodiment, compositions described herein are presented in a pack or dispenser device, such as an FDA approved kit, which contain one or more unit dosage forms containing the active ingredient. In one embodiment, the pack, for example, comprise metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

In one embodiment, it will be appreciated that the compositions described herein are provided to the individual with additional active agents to achieve an improved therapeutic effect as compared to treatment with each agent by itself. In another embodiment, measures (e.g., dosing and selection of the complementary agent) are taken to adverse side effects which are associated with combination therapies.

Additional objects, advantages, and novel features described herein will become apparent to one ordinarily skilled in the art upon examination of the following examples, which are not intended to be limiting. Additionally, each of the various embodiments and aspects described herein as delineated hereinabove and as claimed in the claims section below finds experimental support in the following examples.

### EXAMPLES

Generally, the nomenclature used herein and the laboratory procedures utilized herein include chemical synthesis, molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document.

### Experimental Procedures

### EXAMPLE 1: THE USE OF THE PEPTIDE HUMANIN AND ITS DERIVATIVES TO INHIBIT AND PREVENT NECROSIS

Humanin (HN) is a 24 amino-acid bioactive peptide (APRGFSCLLLLTSEIDLPVKRRA. (SEQ ID NO: 1)). In the present study it was demonstrated that HN and its derivatives are capable of inhibiting necrosis in various cellular systems, and thus can be used as therapeutic treatment for pathological cellular processes which involve necrosis effect in many necrosis related diseases such as but not limited to vascular diseases and trauma. HN and its derivatives were synthesized by solid phase peptide synthesis techniques, purified by reversed-phase HPLC, and their structure (and amino acid sequence) was assessed by analytical reversed-phase HPLC and by MALDI-TOF-mass spectroscopy.

The protective effect of HN and its derivatives was assessed by different methods. The ability of HN and its derivatives to protect cells from necrosis was shown to be independent of the cell death trigger and was observed after necrotizing treatment with KCN or with a combination of oligomycin with staurosporine. Different HN derivatives were able to confer protection. A specific derivative of HN termed HN17 (see Table 1) was able to protect cells from necrosis in all types of cells that were used.

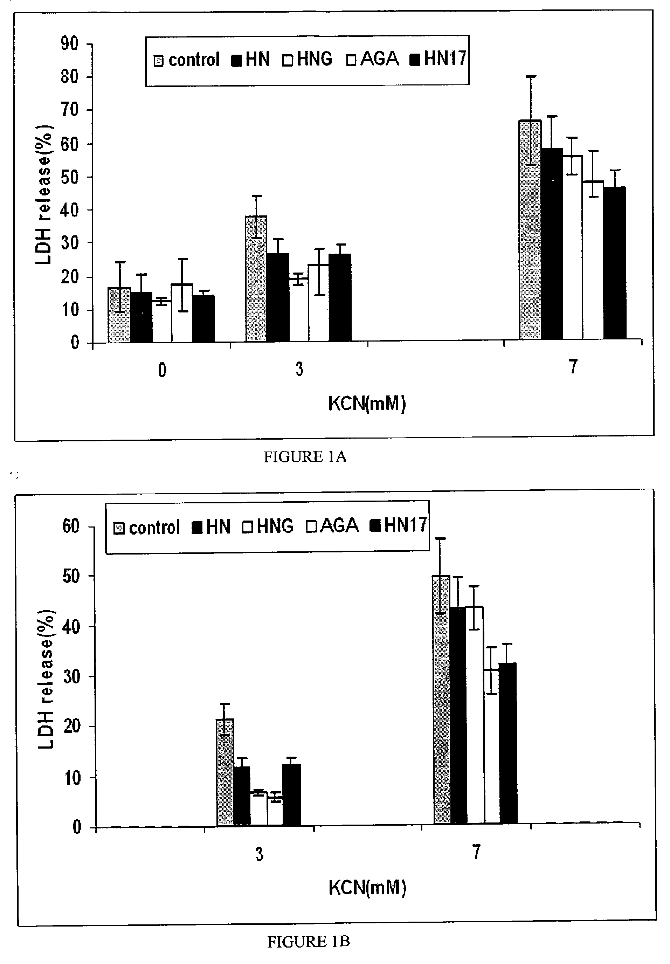

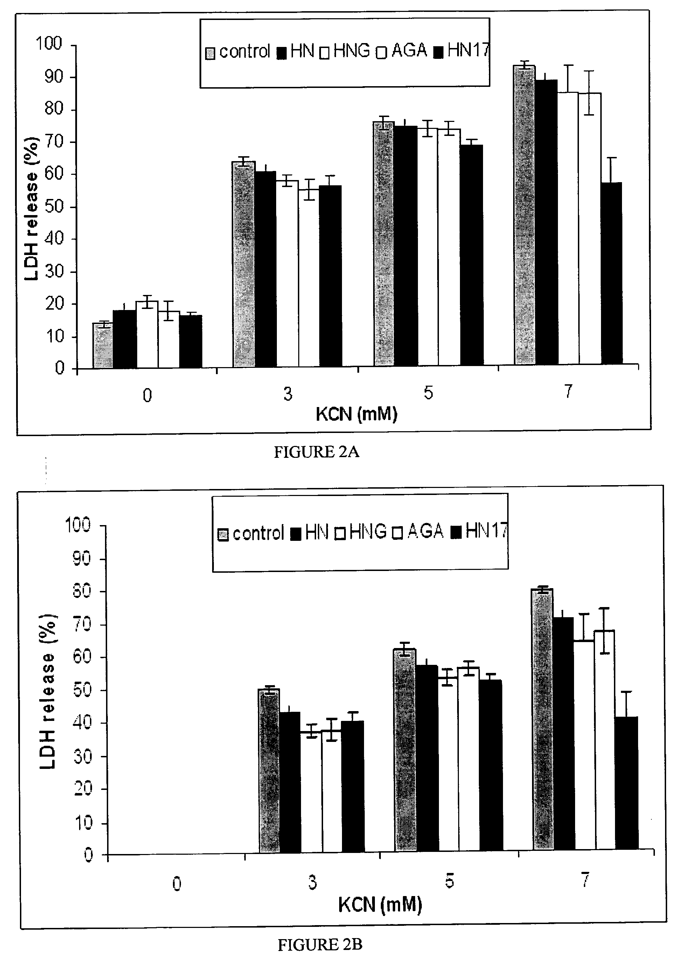

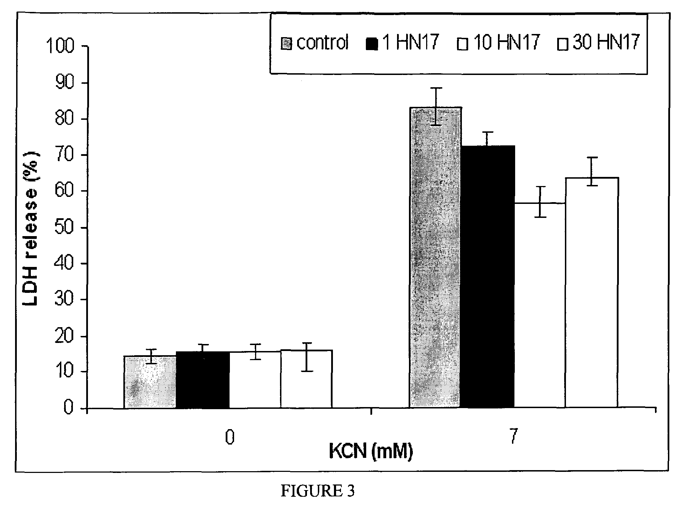

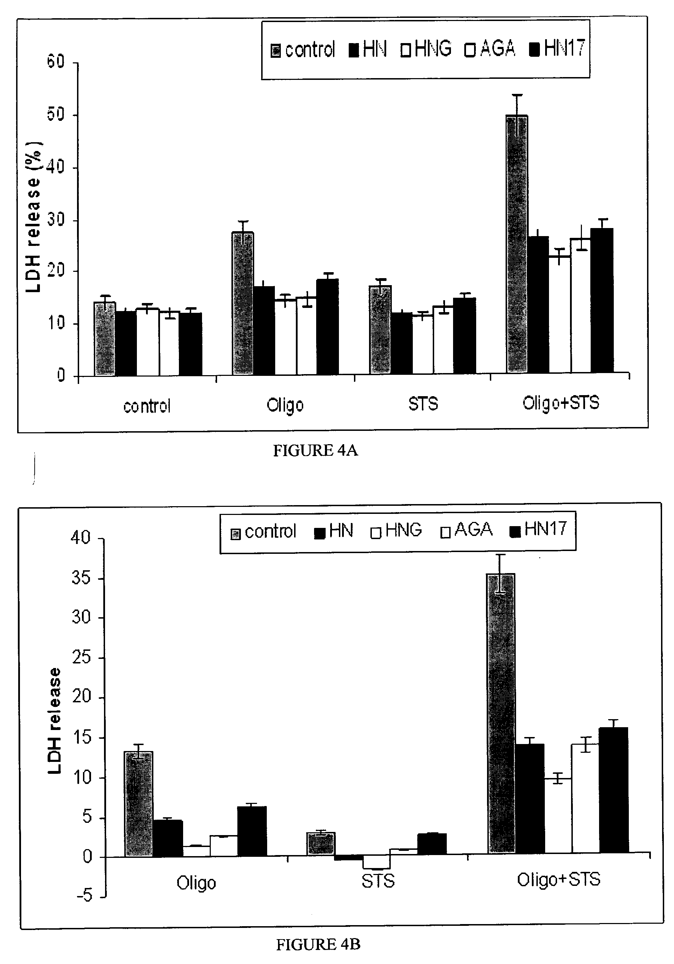

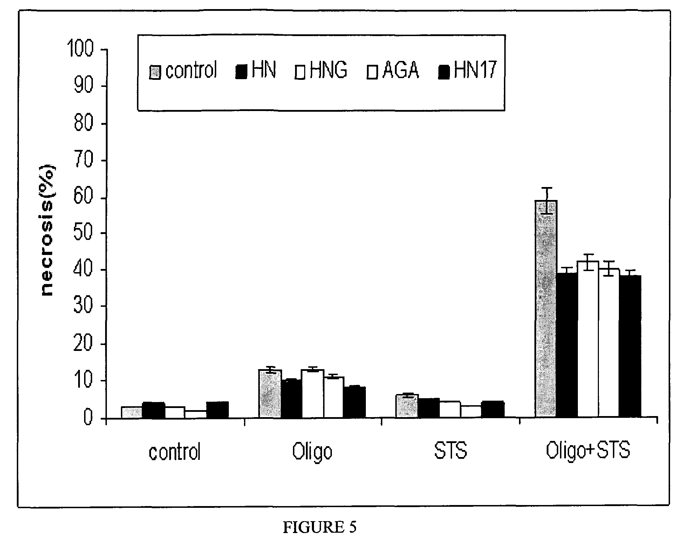

Peptides that were used in the current study are listed in Table 1. As can be seen in Fig. 1, AGA-HNG (AGA) and HN17 conferred protection for U-937 cells against KCN-induced necrotic cell death as measured by LDH release. HN17 confers protection for PC12 cells against KCN-induced necrotic cell death as measured by LDH release (Fig. 2 and Fig. 3). The effects of HN and its derivatives on necrotic cell death induced by staurosporine/ oligomicyn in PC12 cells are depicted in Fig. 4 (by LDH release) and Fig. 5 (ethydium bromide and acridine orange staining). Table 2 hereinbelow summarizes the cell types, as well as the necrosis inducers utilized herein.

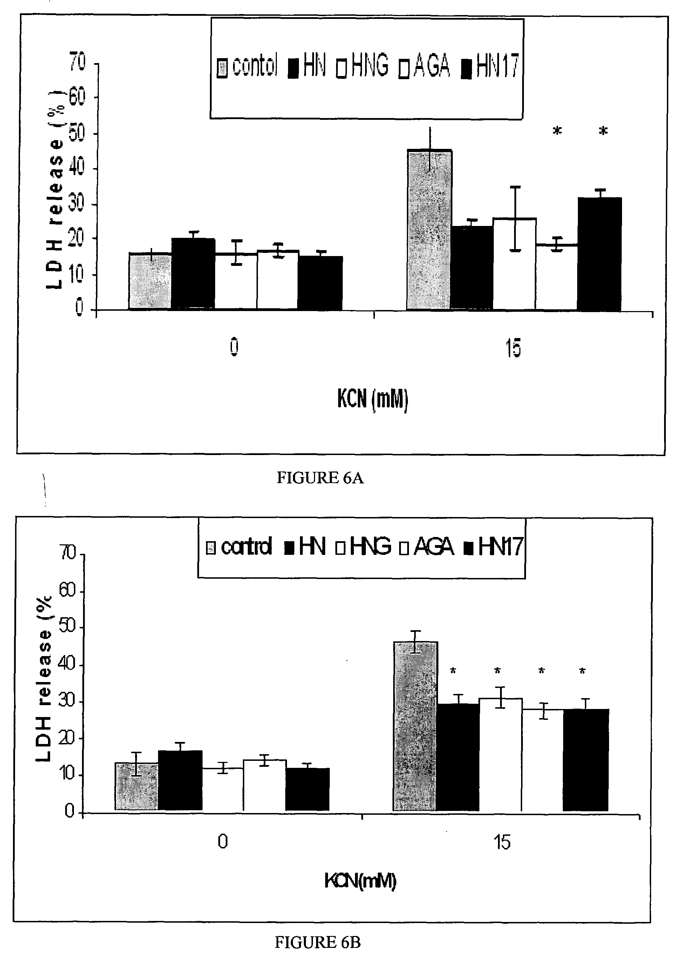

The effect of the peptides was also studied in NSC34 neuromotor cells in models for ALS disease (Fig. 6).

**Table 1: Humanin and derivatives of Humanin that were used herein. Underlined residues represent amino acids that were replaced in the Humanin amino acid sequence**

| Name | Sequence | Remarks |
|---|---|---|
| HN | MAPRGFSCLLLLTSEIDLPVKRRA (SEQ ID NO: 1) | |
| HNG | MAPRGFSCLLLLTGEIDLPVKRRA (SEQ ID NO: 2) | |
| HNA | MAPRGFSALLLLTSEIDLPVKRRA (SEQ ID NO: 7) | Non active used as a control |
| AGA-HNG (AGA) | MAPAGASCLLLLTGEIDLPVKRRA (SEQ ID NO: 3) | |
| HN17 | PRGFSCLLLLTSEIDLP (SEQ ID NO: 4) | |
| HNG17 | PRGFSCLLLLTGEIDLP (SEQ ID NO: 5) | |
| AGA-C8R-HNG17 (HN17) | PAGASRLLLLTGEIDLP (SEQ ID NO: 6) | |

**Table 2: Summary comparison of the effect of HN and its derivatives on cell death induced by different necrotic agents on the various cell lines. Mentioned derivatives have shown protective effect.**

| **Cell death inducer/ Cell line** | **U937** | **PC12** | **NSC34^{a}** | **HL1** |
|---|---|---|---|---|
| **KCN** | AGA and HN17 | HN17 | All derivatives | AGA and HN17 |
| **Staurosporine/ oligomycin** | - | All derivatives | - | - |

| | | | | |
|---|---|---|---|---|
| ^{a} NSC34 cells includes cells without plasmid and SOD1 mutant and wild type plasmid transfected cells. | | | | |

Thus the peptides are effective in both preventing and inhibiting necrosis.

### EXAMPLE 2: THE SYNERGISTIC EFFECT OF HUMANIN WITH ELASTASE INHIBITOR III

In these set of experiments it was shown that a composition comprising a combination of: (1) Humanin or a derivative thereof along with (2) a necrosis inhibitor such a neutrophil Elastase inhibitor (Elastase inhibitor III), is efficient in treating a patient suffering from a disease characterized by necrotic tissue. These experiments provide that Humanin or a derivative thereof is/are favorably and unexpectedly combined with a necrosis inhibitor at a low concentration in which neither of them separately has any effect. Thus a proof for a synergistic effect of a composition comprising both (1) Humanin and a derivative thereof; and (2) a neutrophil Elastase inhibitor (Elastase inhibitor III), in inhibiting necrotic cell death is provided. The effect of the synergistic combination is at a low concentration in which neither of: (1) Humanin and a derivative thereof; or (2) a neutrophil Elastase inhibitor (Elastase inhibitor III) has any effect, but when administered together have a synergistic effect in inhibiting cell death by necrosis.

### U937 Cell Line

The U937 cells are p53 minus monocytic cell line. The cells were grown at 37°C in the presence of 5% CO₂ in RPMI-1640 medium supplemented with 10% heat-inactivated fetal bovine serum (FCS), 2 Mm glutamine, 100 µg/ml penicillin, and 100 µg/ml streptomycin. The cells were split every third day.

### KCN induced necrosis

U937 cells were cultured in complete RPMI-1640 medium, washed once and seeded at 4.5x10⁵ cells/ml in glucose free medium 1 hour before treatment. Humanin or its derivatives of SEQ ID MOs 1-6 and Elastase inhibitor III (EI-III) and vehicle were added 30 minutes before the addition of KCN (15mM). Following the treatment, the cells were incubated for 7 hours and cell death was measured via the LDH release assay.

### LDH release assay

The amount of LDH released from lysed cells is a sensitive measure of cell death. In this study, necrotic cell death was measured in 96-well plates using Promega's CytoTox 96 Non-Radioactive Cytotoxicity Assay kit, which accurately and rapidly measures cell death by quantitating the release of lactate dehydrogenase (LDH), a stable cytosolic enzyme from lysed cells.

At the end of each experiment, the cells were centrifuged at 240xg for 10 minutes at room temperature. Next, the supernatant was collected and 50µL aliquots were taken for the LDH release assay. The LDH content from cells lysed in 0.1% Tryton X-100 for 10-15 minutes was used as a measure for total LDH content. The LDH released in the treatment (after subtracting the blank measure) was used for measuring necrotic cell death as a percentage from total LDH. A measurement of the absorbance value at 490nm was preformed by ELISA READER, 30 minutes after the addition of the LDH reaction solution.

### Medium

RPMI-1640 medium was purchased from Gibco (Rhenium, Israel), glucose free RPMI-1640 medium, fetal calf serum (FCS), penicillin/streptomycin solution (penicillin 10000units/ml; streptomycin 10mg/ml), L-glutamine solution 200 mM, phosphate-buffered saline solution (PBS) were purchased from Biological Industries (Beth Haemek, Israel). CytoTox 96 Non-Radioactive Cytotoxicity Assay kit was purchased from Promega (Beth Haemek, Israel). Elastase Inhibitor III was purchased from Calbiochem (Mercury, Israel). Humanin and its derivatives (SEQ ID NOs: 1-6) were synthesized at the Department of Desalination and Water Treatment (Zuckerberg Institute for Water Research, The J. Blaustein Institutes for Desert Research, Ben-Gurion University of the Negev, Sede Boqer Campus, Israel).

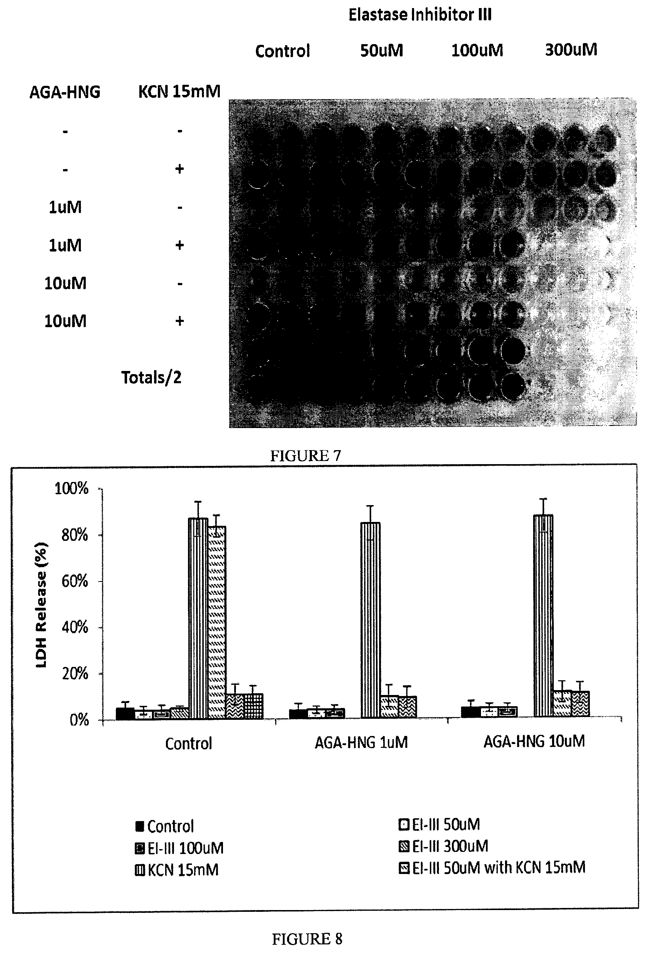

The results of Fig. 7 show that treating U937cells with KCN at 15mM induced 81%cell death by necrosis (Fig. 8). However, treatment with Elastase inhibitor III (EI-III) at 300µM and 100µM inhibited cell death by necrosis by 75%. Treatment with EI-III at 50µM had no impact on necrotic cell death induced by KCN. Treatment with AGA-HNG (SEQ ID NO: 3) at 1µM and 10µM also had no impact on necrotic cell death induced by KCN. However, treatment with the combination of 1µM or 10µM AGA-HNG and 50µM EI-III inhibited cell death by necrosis by 74%.

Thus, the combination therapy of as little as 1µM AGA-HNG with 50µM EI-III inhibited necrotic cell death induced by KCN at the same levels that large amount of 300µM EI-III inhibited necrotic cell death induced by KCN. This shows a synergistic effect of EI-III and Humanin derivatives. The Humanin derivatives unexpectedly and dramatically potentiate EI-III thus providing a synergistic combination. This unexpected result enables the use of this combination therapy while minimizing undesired effects associated with large quantities of either or both EI-III and Humanin derivatives.

### EXAMPLE 3: THE SYNERGISTIC EFFECT OF HUMANIN WITH MIMOSINE

In these set of experiments it was shown that a composition comprising a combination of: (1) Humanin or a derivative thereof along with (2) a necrosis inhibitor such Mimosine, is efficient in treating a patient suffering from a disease characterized by necrotic tissue. These experiments provide that Humanin or a derivative thereof is/are favorably and unexpectedly combined with a necrosis inhibitor at a low concentration in which neither of them separately has any effect. Thus a proof for a synergistic effect of a composition comprising both (1) Humanin and a derivative thereof; and (2) Mimosine, in inhibiting necrotic cell death is provided. The effect of the synergistic combination is at a low concentration in which neither of: (1) Humanin and a derivative thereof; or (2) Mimosine has any effect, but when administered together have a synergistic effect in inhibiting cell death by necrosis.

### U937 Cell Line

The U937 cells are p53 minus monocytic cell line. The cells were grown at 37°C in the presence of 5% CO₂ in RPMI-1640 medium supplemented with 10% heat-inactivated fetal bovine serum (FCS), 2 Mm glutamine, 100 µg/ml penicillin, and 100 µg/ml streptomycin. The cells were split every third day.

### KCN induced necrosis

U937 cells were cultured in complete RPMI-1640 medium, washed once and seeded at 4.5x10⁵ cells/ml in glucose free medium 1 hour before treatment. Humanin or its derivatives of SEQ ID MOs 1-6 and Mimosine and vehicle were added 30 minutes before the addition of KCN (5mM). Following the treatment, the cells were incubated for 7 hours and cell death was measured via the LDH release assay.

### LDH release assay

The amount of LDH released from lysed cells is a sensitive measure of cell death. In this study, necrotic cell death was measured in 96-well plates using Promega's CytoTox 96 Non-Radioactive Cytotoxicity Assay kit, which accurately and rapidly measures cell death by quantitating the release of lactate dehydrogenase (LDH), a stable cytosolic enzyme from lysed cells.

At the end of each experiment, the cells were centrifuged at 240xg for 10 minutes at room temperature. Next, the supernatant was collected and 50µL aliquots were taken for the LDH release assay. The LDH content from cells lysed in 0.1% Tryton X-100 for 10-15 minutes was used as a measure for total LDH content. The LDH released in the treatment (after subtracting the blank measure) was used for measuring necrotic cell death as a percentage from total LDH. A measurement of the absorbance value at 490nm was preformed by ELISA READER, 30 minutes after the addition of the LDH reaction solution.

### Medium

RPMI-1640 medium was purchased from Gibco (Rhenium, Israel), glucose free RPMI-1640 medium, fetal calf serum (FCS), penicillin/streptomycin solution (penicillin 10000units/ml; streptomycin 10mg/ml), L-glutamine solution 200 mM, phosphate-buffered saline solution (PBS) were purchased from Biological Industries (Beth Haemek, Israel). CytoTox 96 Non-Radioactive Cytotoxicity Assay kit was purchased from Promega (Beth Haemek, Israel). Elastase Inhibitor III was purchased from Calbiochem (Mercury, Israel). Humanin and its derivatives (SEQ ID NOs: 1-6) were synthesized at the Department of Desalination and Water Treatment (Zuckerberg Institute for Water Research, The J. Blaustein Institutes for Desert Research, Ben-Gurion University of the Negev, Sede Boqer Campus, Israel).

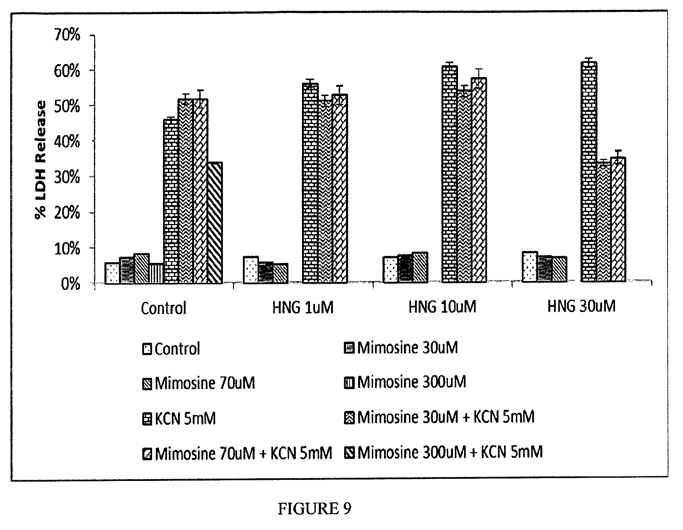

The results provided in Fig. 9 show that the treating U937 cells with KCN at 5mM induced cell death by necrosis to 40% of the cells. Treatment with mimosine at 300µM inhibited cell death by necrosis by 12%. Treatment with mimosine at 30µM and 70 µM was refractory-caused no inhibition of necrotic cell death. Treatment with HNG (SEQ ID NO: 3) at 1µM, 10µM and 30µM was also refractory-caused no inhibition of necrotic cell death. However, treatment of both HNG at 30µM and mimosine at 30µM or 70 µM caused significant inhibition of necrotic cell death by 18% in average

Thus, this combination therapy inhibited necrotic cell death induced by KCN at the same levels that large and toxic amount of mimosine or HNG inhibited necrotic cell death induced by KCN. This shows a synergistic effect of mimosine and Humanin derivatives. The Humanin derivatives unexpectedly and dramatically potentiate mimosine thus providing a synergistic combination. This unexpected result enables the use of this combination therapy while minimizing undesired effects associated with large quantities of either or both mimosine and Humanin derivatives.

Mimosine, a non-protein amino acid, is mainly known for its action as a reversible inhibitor of DNA replication and apoptosis inducer; therefore, it has been widely used as a cell cycle synchronizing agent. The present results demonstrate that while mimosine 30µM or 70 µM by itself did not inhibit necrotic cell death induced by KCN, the treatment of HNG at 30 µM with mimosine at 30µM was enough to inhibit necrotic cell death at the same level that mimosine at 300µM inhibits. This shows a synergistic effect of Humanin and its derivatives of SEQ ID NOs: 1-6 with mimosine to inhibit necrotic cell death induced by KCN. Thus synergistic combinations for treating diseases caused by or associated with necrosis, are provided.

## Claims

1. A composition comprising: (a) an inhibitor of neutrophil Elastase; and (b) Humanin or a peptide comprising an amino acid sequence selected from the group consisting of SEQ ID NOs: 1-6.

2. A composition comprising: (a) Mimosin; and (b) Humanin or a peptide comprising an amino acid sequence selected from the group consisting of SEQ ID NOs: 1-6.

3. A peptide comprising an amino acid sequence selected from the from the group consisting of SEQ ID NOs: 1-6, for use in the treatment of a disease **characterized by** tissue necrosis in a subject, wherein an effective amount of said peptide is to be administered to said subject and wherein necrosis is thereby inhibited.

4. The peptide for use according to claim 3, wherein said disease is selected from the group comprising: a neurodegenerative disorder, leukemia, lymphoma, neonatal respiratory distress, asphyxia, incarcerated hernia, diabetes, tuberculosis, endometriosis, vascular dystrophy, psoriasis, cold injury, an iron-load complication, complication of steroid treatment, ischemic heart disease, reperfusion injury, cerebrovascular disease, gangrene, a pressure sore, pancreatitis, hepatitis, hemoglobinuria, sepsis, burns, hyperthermia, Crohn's disease, celiac disease, compartment syndrome, necrotizing procolitis, cystic fibrosis, rheumatoid arthritis, nephrotoxicity, multiple sclerosis, spinal cord injury, glomerulonephritis, muscular dystrophy, degenerative arthritis, tyromesia, metabolic inherited disease, mycoplasmal disease, anthrax infection, bacterial infection, viral infection, Anderson disease, congenital mitochondrial disease, phenylketonuria, placental infarct, syphilis, asceptic necrosis, avascular necrosis, alcoholism, necrosis associated with administration and/or self-administration with, and/or exposure to drugs, chemical toxins, cyanide, methanol, ethylene glycol, mustard gas, agrochemicals, organophosphates and necrosis associated with aging.

5. The peptide for use according to claim 3, wherein said tissue is blood or neuronal.

6. A peptide comprising an amino acid sequence selected from the from the group consisting of SEQ ID NOs: 1-6, for use in treating an ischemic heart disease in a subject, wherein an effective amount of said peptide is to be administered to said subject and wherein necrosis is thereby inhibited.

7. An in vitro method for inhibiting necrosis in a cell, comprising the step of contacting said cell with a composition comprising an effective amount of a peptide comprising an amino acid sequence selected from the group consisting of SEQ ID NOs: 1-6, thereby inhibiting necrosis in a cell.

8. The method of claim 7, wherein said cell is a blood cell or a neuronal cell.

9. The method of claim 7, wherein said composition further comprises an inhibitor of neutrophil Elastase.

10. A peptide comprising an amino acid sequence selected from the from the group consisting of SEQ ID NOs: 1-6, for use in the prevention of pancreatitis resulting from an abdominal surgical procedure in a subject, wherein an effective amount of said peptide is to be administered to said subject and wherein necrosis is thereby inhibited.

## Patentansprüche

1. Zusammensetzung, die aufweist: (a) einen Inhibitor neurophiler Elastase und (b) Humanin oder ein Peptid, das eine Aminosäuresequenz umfasst, die unter den SEQ ID NO: 1-6 ausgewählt ist.

2. Zusammensetzung, die aufweist: (a) Mimosin und (b) Humanin oder ein Peptid, das eine Aminosäuresequenz umfasst, die unter den SEQ ID NO: 1-6 ausgewählt ist.

3. Peptid, das eine Aminosäuresequenz umfasst, die unter den SEQ ID NO: 1-6 ausgewählt ist, zur Verwendung bei der Behandlung einer durch Gewebenekrose gekennzeichneten Erkrankung bei einem Subjekt, wobei dem Subjekt eine wirksame Menge des Peptids zu verabreichen ist und wobei die Nekrose dadurch inhibiert wird.

4. Peptid zur Verwendung nach Anspruch 3, wobei die Erkrankung ausgewählt ist unter: neurodegenerativen Erkrankungen, Leukämie, Lymphom, neonataler Atemnot, Asphyxie, inkarzerierter Hernie, Diabetes, Tuberkulose, Endometriose, vaskulärer Dystrophie, Psoriasis, Kälteschäden, Eisen-Aufnahme-Komplikationen, Komplikationen einer Steroidbehandlung, ischämischer Herzkrankheit, Reperfusionsschäden, zerebrovaskulären Krankheiten, Gangrän, Druckstellen, Pankreatitis, Hepatitis, Hämoglobinurie, Sepsis, Verbrennungen, Hyperthermie, Morbus Crohn, Zöliakie, Kompartmentsyndrom, nekrotisierender Prokolitis, zystischer Fibrose, rheumatoider Arthritis, Nephrotoxizität, Multipler Sklerose, Rückenmarksverletzung, Glomerulonephritis, Muskeldystrophie, degenerativer Arthritis, Tyromesia, angeborenen Stoffwechselerkrankungen, Mykoplasmen-Erkrankung, Anthrax-Infektion, bakterieller Infektion, Virusinfektion, Andersen-Syndrom, kogenitalen mitochondrialen Erkrankungen, Phenylketonurie, Placenta-Infarkt, Syphilis, aseptischer Nekrose, avaskulärer Nekrose, Alkoholismus, Nekrose, die mit einer Verabreichung und/oder Eigenverabreichung und/oder Einwirkung von Drogen, chemischen Giftstoffen, Cyanid, Methanol, Ethylenglycol, Senfgas, Agrochemikalien und Organophosphaten zusammenhängt, und mit Alterung einhergehender Nekrose.

5. Peptid zur Verwendung nach Anspruch 3, wobei es sich bei dem Gewebe um Blut oder Neuronen handelt.

6. Peptid, das eine Aminosäuresequenz umfasst, die unter den SEQ ID NO: 1-6 ausgewählt ist, zur Verwendung bei der Behandlung einer ischämischen Herzerkrankung bei einem Subjekt, wobei dem Subjekt eine wirksame Menge des Peptids zu verabreichen ist und wobei die Nekrose dadurch inhibiert wird.

7. In vitro-Verfahren zur Inhibierung von Nekrose in einer Zelle, das den Schritt umfasst, die Zelle mit einer Zusammensetzung in Kontakt zu bringen, die ein Peptid, das eine Aminosäuresequenz umfasst, die unter den SEQ ID NO: 1-6 ausgewählt ist, in einer wirksamen Menge enthält, wobei die Nekrose in der Zelle dadurch inhibiert wird.

8. Verfahren nach Anspruch 7, wobei es sich bei der Zelle um eine Blutzelle oder Nervenzelle handelt.

9. Verfahren nach Anspruch 7, wobei die Zusammensetzung ferner einen Inhibitor neurophiler Elastase enthält.

10. Peptid, das eine Aminosäuresequenz umfasst, die unter den SEQ ID NO: 1-6 ausgewählt ist, zur Verwendung bei der Vorbeugung von Pankreatis, die aus einem abdominalen chirurgischen Eingriff resultiert, bei einem Subjekt, wobei dem Subjekt eine wirksame Menge des Peptids zu verabreichen ist und wobei die Nekrose dadurch inhibiert wird.

## Revendications

1. Composition comprenant : (a) un inhibiteur de l'élastase neutrophile ; et (b) l'humanine ou un peptide comprenant une séquence d'aminoacides choisie dans le groupe consistant en les SEQ ID N° 1 à 6.

2. Composition comprenant : (a) la mimosine ; et (b) l'humanine ou un peptide comprenant une séquence d'aminoacides choisie dans le groupe consistant en les SEQ ID N° 1 à 6.

3. Peptide comprenant une séquence d'aminoacides choisie dans le groupe consistant en les SEQ ID N° 1 à 6, pour une utilisation dans le traitement d'une maladie **caractérisée par** une nécrose tissulaire chez un sujet, où une quantité efficace dudit peptide doit être administrée audit sujet et où la nécrose est ainsi inhibée.

4. Peptide pour une utilisation selon la revendication 3, dans lequel ladite maladie est choisie dans le groupe comprenant : un trouble neurodégénératif, la leucémie, le lymphome, la détresse respiratoire du nouveau-né, l'asphyxie, la hernie incarcérée, le diabète, la tuberculose, l'endométriose, la dystrophie vasculaire, le psoriasis, des lésions dues au froid, une complication liée à la charge en fer, une complication d'un traitement stéroïdien, une maladie cardiaque ischémique, une lésion de reperfusion, une maladie cérébrovasculaire, la gangrène, une escarre de décubitus, la pancréatite, l'hépatite, l'hémoglobinurie, une sepsie, des brûlures, l'hyperthermie, la maladie de Crohn, une maladie coeliaque, le syndrome compartimental, la proctite nécrosante, la fibrose kystique, la polyarthrite rhumatoïde, la néphrotoxicité, la sclérose en plaques, une lésion de la moelle épinière, la glomérulonéphrite, la dystrophie musculaire, l'arthrite dégénérative, la tyromesia, une maladie héréditaire métabolique, une maladie mycoplasmique, une infection par l'anthrax, une infection bactérienne, une infection virale, la maladie d'Anderson, la maladie mitochondriale congénitale, la phénylcétonurie, l'infarctus placentaire, la syphilis, la nécrose aseptique, la nécrose avasculaire, l'alcoolisme, la nécrose associée à l'administration et/ou à l'auto-administration de médicaments, de toxines chimiques, du cyanure, du méthanol, de l'éthylèneglycol, du gaz moutarde, de produits agrochimiques, d'organophosphates et/ou à l'exposition à ceux-ci et la nécrose associée au vieillissement.

5. Peptide pour une utilisation selon la revendication 3, dans lequel ledit tissu est un tissu sanguin ou un tissu neuronal.

6. Peptide comprenant une séquence d'aminoacides choisie dans le groupe consistant en les SEQ ID N° 1 à 6, pour une utilisation dans le traitement d'une maladie cardiaque ischémique chez un sujet, où une quantité efficace dudit peptide doit être administrée audit sujet et où la nécrose est ainsi inhibée.

7. Procédé in vitro d'inhibition de la nécrose dans une cellule, comprenant l'étape de mise en contact de ladite cellule avec une composition comprenant une quantité efficace d'un peptide comprenant une séquence d'aminoacides choisie dans le groupe consistant en les SEQ ID N° 1 à 6, inhibant ainsi la nécrose dans une cellule.

8. Procédé de la revendication 7, dans lequel ladite cellule est une cellule sanguine ou une cellule neuronale.

9. Procédé de la revendication 7, dans lequel ladite composition comprend en outre un inhibiteur de l'élastase neutrophile.

10. Peptide comprenant une séquence d'aminoacides choisie dans le groupe consistant en les SEQ ID N° 1 à 6, pour une utilisation dans la prévention de la pancréatite résultant d'une intervention chirurgicale abdominale chez un sujet, où une quantité efficace dudit peptide doit être administrée audit sujet et où la nécrose est ainsi inhibée.


### SEQUENCE LISTING

<110> Ben Gurion university of the Negev Research and Development Authority Parola, Abraham Dahan, Avraham Kasher, Ron Nathan, Ilana
<120> METHODS FOR INHIBITING NECROSIS
<130> P-73957-PC
<150> 61/307,647
   <151> 2010-02-24
<160> 7
<170> PatentIn version 3.5
<210> 1
   <211> 24
   <212> PRT
   <213> Homo sapiens
<400> 1
   Met Ala Pro Arg Gly Phe Ser Cys Leu Leu Leu Leu Thr Ser Glu Ile
   1               5                   10                  15
   Asp Leu Pro Val Lys Arg Arg Ala
               20
<210> 2
   <211> 24
   <212> PRT
   <213> Homo sapiens
<400> 2
   Met Ala Pro Arg Gly Phe Ser Cys Leu Leu Leu Leu Thr Gly Glu Ile
   1               5                   10                  15
   Asp Leu Pro Val Lys Arg Arg Ala
               20
<210> 3
   <211> 24
   <212> PRT
   <213> Homo sapiens
<400> 3
   Met Ala Pro Ala Gly Ala Ser Cys Leu Leu Leu Leu Thr Gly Glu Ile
   1               5                   10                  15
   Asp Leu Pro Val Lys Arg Arg Ala
               20
<210> 4
   <211> 17
   <212> PRT
   <213> Homo sapiens
<400> 4
   Pro Arg Gly Phe Ser Cys Leu Leu Leu Leu Thr Ser Glu Ile Asp Leu
   1               5                   10                  15
   Pro
<210> 5
   <211> 17
   <212> PRT
   <213> Homo sapiens
<400> 5
   Pro Arg Gly Phe Ser Cys Leu Leu Leu Leu Thr Gly Glu Ile Asp Leu
   1               5                   10                  15
   Pro
<210> 6
   <211> 17
   <212> PRT
   <213> Homo sapiens
<400> 6
   Pro Ala Gly Ala Ser Arg Leu Leu Leu Leu Thr Gly Glu Ile Asp Leu
   1               5                   10                  15
   Pro
<210> 7
   <211> 24
   <212> PRT
   <213> Homo sapiens
<400> 7
   Met Ala Pro Arg Gly Phe Ser Ala Leu Leu Leu Leu Thr Ser Glu Ile
   1               5                   10                  15
   Asp Leu Pro Val Lys Arg Arg Ala
               20